Europäisches Patentamt

European Patent Office

Office européen des brevets

(11) Publication number: 0 328 279 A1

# EUROPEAN PATENT APPLICATION

(21) Application number: 89300845.8

(22) Date of filing: 27.01.89

(51) Int. Cl.⁴: C07C 25/02 , C07C 17/12 , C09K 7/06

(30) Priority: 06.02.88 GB 8802732

(43) Date of publication of application:
16.08.89 Bulletin 89/33

(84) Designated Contracting States:
BE CH DE ES FR GB IT LI NL SE

(71) Applicant: The British Petroleum Company p.l.c.
Britannic House Moor Lane
London EC2Y 9BU(GB)

(72) Inventor: Butters, Michael
Kimben Mongeham Road Ripple
Near Deal Kent CT14 8JW(GB)

(74) Representative: MacLeod, Malcolm et al
BP INTERNATIONAL LIMITED Patents
Division Chertsey Road
Sunbury-on-Thames Middlesex TW16 7LN(GB)

(54) Preparation of halogenated organic compounds.

(57) A mixture of brominated aromatic hydrocarbons is produced by a two-stage process involving reacting an aromatic hydrocarbon and bromine in a first stage to give a penta-bromo derivative, and then, in the presence of the first stage product, reacting a further quantity of aromatic hydrocarbon and bromine to give tri-bromo derivative or a mixture of di- and tri-bromo derivatives with the latter predominating.

The mixture may have 40-95 mole per cent of tri- or di- and tri-bromo derivatives, and 40-5 mole per cent of penta-bromo derivative with not more than 2 mole per cent of tetra-bromo derivatives.

The mixture is suitable as a well bore fluid, and can be varied in composition to give products with a range of specific gravities for that purpose. The substantial absence of tetra-bromo derivatives means that the products can be used without toxicological problems.

## PREPARATION OF HALOGENATED ORGANIC COMPOUNDS

This invention relates to a process for the halogenation of organic compounds, to the products of such a process, and to their use as solids-free, non-aqueous well-bore fluids of high specific gravity which can be used during or after drilling to complete and/or treat a production or injection well.

The fluids are useful as completion fluids or work-over fluids, jointly termed well-bore fluids, where high stability, low corrosion and absence of solidification are desired.

The term "solids-free" is applied to the basic well-bore fluid having the desired specific gravity. This term is understood in the art to mean that no solid weighting agent is employed. In certain cases, however, solid additives may be added to the well-bore fluid for specific purposes.

Examples of well-bore fluids include drill-in fluids, fracturing fluids, perforating fluids, gravel packing fluids and packer fluids.

After an oil or gas well has been drilled, the casing is perforated to provide access through the casing to the earth formation containing the hydrocarbons to be recovered. This can be done by exploding shaped charges of various types in the casing or by mechanical punch-type casing perforators. In any event, upon perforating the casing, the interior of the well is subjected to the earth formation pressure and requires a counter balanced hydrostatic pressure of fluid in the well to prevent loss of control of the well. In practice, the hydrostatic pressure in the well is usually maintained somewhat higher than that of the earth formation, and some of the fluid in the well often flows through the perforations into the earth formation.

In such an instance, it is undesirable to employ drilling muds as the well-bore fluid. The muds, with their solid constituents, tend to plug perforations and, if they enter the earth formation, they can interfere with the proper recovery of the desired hydrocarbon from the reservoir, particularly in sandy formations. In order to avoid such problems, it is common to use a solids-free completion fluid which is maintained in the well to balance the pressure exerted by the earth formation.

Another use for such a fluid, in this context termed "packer fluid", is to exert a hydrostatic head on an annular packer to ensure that the produced oil or gas only issues from the tubing in the well under the control of the well operator. In practice, the packer is placed in the annular space between the casing and tubing, fluid tight, so that the formation products such as gas or oil, are prevented from escaping from the well except through the tubing. This annular space above the packer is then filled with a packer fluid to maintain a hydrostatic pressure on the up-bore or top side of the packer which is about the same, or perhaps slightly greater, than the pressure of the producing formation. By employing such a fluid the formation products produce the same, or slightly less, pressure on the other side or down-bore side of the packer as the added fluid does on the opposite side of the packer. Thus, the removal of any substantial differential pressure across the packer minimises any tendency for the formation products to bleed or leak around the packer.

In order for a well-bore fluid to be useful in these and other applications, the fluid must have sufficient specific gravity to exert the required hydrostatic pressure, and, preferably, its specific gravity should be capable of being varied to exert the desired amount of hydrostatic pressure to balance the pressure exerted by the earth formation. The hydrostatic pressure of the fluid is based upon the height of the column of fluid in the well and its specific gravity. Since the well depth, and consequently the height of the column of fluid in the well is fixed, the only remaining variable, namely, the specific gravity of the fluid, should be capable of being varied to meet the needs of the hydrostatic pressure required downhole.

This is currently achieved by one of two means. Frequently, dense particulate materials such as barytes or calcium carbonate are suspended in a carrier fluid. A major disadvantage of this method is the migration of these solids into the pay-zone leading to an impairment in hydrocarbon recovery. To alleviate this problem, dense, solids-free, brine solutions of various formulations have been proposed.

Well-bore fluids should be non-corrosive to the ferrous metal tubing and pipes which they contact for prolonged periods. Once a producing well is established and pipe, packer and completion fluid have been installed, replacement of any part of the pipe string, because of corrosion by the completion fluid, amounts to a major undertaking, requiring shut down of the well and a costly and extended period for removal and replacement of the pipe string. In addition, if the corrosion is severe and rapid, loss of control of the well due to pipe rupture is a serious possibility.

Well-bore fluids can be (1) water based, e.g. brines, (2) invert emulsions or (3) oil based systems.

Water based systems are frequently employed, particularly clear brines, but they suffer from the disadvantages that they are sometimes toxic (and therefore require special handling procedures), corrosive (and require the use of well liners and/or corrosion inhibitors), and can recrystallise and show incompatability with reservoir fluids.

They are also subject to foaming problems and are hygroscopic. Absorption of water leads to loss of specific gravity and further control problems. Yet another disadvantage is their tendency to attack elastomeric seals in well-bore equipment.

Invert emulsion fluids can be weighted with acid soluble materials such as calcium carbonate and show little reaction with reservoir clays. The surfactants used to generate the invert emulsion, can, however, damage pay-zone formations by wettability changes.

Damage to the formation is a particularly acute problem in many wells. This can be caused by solids invasion from solid particles in the well-bore fluid, such as barytes or clay, or fluid invasion by the fluid itself. This can give rise to dispersion and migration of reservoir clays, emulsion blocking and scale precipitation.

Clean crude oil is naturally the least damaging completion fluid to be placed across an oil-bearing formation. However, its use has been seriously limited due to the difficulty in suspending weighting agents in it, and, even if this is overcome, the latter can give rise to problems outlined above.

Our copending European Patent Application No. 0247801-A describes and claims a method for the completion or work-over of a well which method comprises the step of using a solids-free, non-aqueous well-bore fluid comprising a halogenated organic compound as a completion or work-over fluid, the fluid having a specific gravity in the range 0.9 to 2.3, preferably 1.5 to 2.2.

The preferred organic compounds are brominated organic compounds, brominated aromatic and alkyl aromatic compounds being particularly suitable, e.g. brominated ethyl benzene and cumene.

However, amongst the brominated ethyl benzenes and cumenes which satisfy the technical requirements for dense, well-bore fluids (e.g. being homogeneous liquids at -10°C with a viscosity not greater than 600 cp. and having good high temperature stability at 200°C under a range of pressures) only dibromoethyl benzenes, tribromoethyl benzenes, pentabromoethyl benzene and tribromocumenes are currently listed on TOSCA or EINECS environmental toxicology registers, and are therefore available for permitted use without further extensive toxicological testing.

We have now devised a process for the bromination of an aromatic compound, which is selective to a product in which di-, tri-, and pentabromo derivatives predominate, without the need for blending individual components.

Thus according to the present invention there is provided a two-stage process for the bromination of an aromatic hydrocarbon which process comprises

(a) initially reacting the aromatic compound with bromine in a molar ratio of bromine to aromatic compound in the range 30:1 to 5:1, preferably 10:1 to 7:1, most preferably 8.5:1, in the presence of a bromination catalyst to convert the compound substantially to the pentabromo-derivative and

(b) subsequently reacting, in the presence of the products from stage (a), a further quantity of aromatic compound in a ratio to the initial quantity in the range 100:1 to 0.1:1, preferably 20:1 to 5:1, most preferably 9:1, with a further quantity of bromine in a molar ratio of bromine to aromatic compound in the range 10:1 to 1:1, preferably 5:1 to 2:1, most preferably 2.5:1, in the presence of the bromination catalyst, for a time sufficient to convert the reactants of stage (b) to tribromo derivatives or a mixture of tribromo and dibromo derivatives in which tribromo-derivatives predominate.

The higher the ratio of bromine to the aromatic compound, the shorter will be the reaction time in stage (b), and vice versa.

In the second stage (b), the additional quantity of aromatic compound should be added at a rate such that formation of tetrabromo-and pentabromo-derivatives (by reaction with excess bromine remaining from the first stage) is minimised. It should be added as quickly as possible consistent with maintaining a constant temperature and avoiding a substantial exotherm, and minimising the production of tetra-and penta-bromo derivatives.

The second stage (b) can be carried out in several different ways.

The preferred route involves adding the reaction product of stage (a) to the second portion of the aromatic compound contained in a second reactor and subsequently adding more bromine.

Alternatively, a single reactor may be employed and the second portion of the aromatic compound added to the reaction product of stage (a), followed by more bromine.

Less preferred routes include adding the reaction product of stage (a) to a mixture of the second portion of the aromatic compound and bromine, and adding a mixture of the second portion of the aromatic compound and bromine to the reaction product of stages (a).

The second phase of bromine addition may thus take place concurrently with the addition of the aromatic compound, but preferably all the latter is added before any of the former.

The aromatic compound is preferably an alkyl aromatic hydrocarbon, most preferably ethylbenzene. Cumene is also suitable.

It is also possible to use different compounds in the different stages. For example, ethylbenzene may be brominated in the first stage and cumene in the second, in the presence of the brominated ethyl benzene. The resulting mixture of products may have improved low temperature properties.

Because, as previously stated, tetrabromo ethylbenzenes are not listed on toxicology registers, it is desirable, in the case of brominated ethylbenzenes that the final product is not contaminated by more than 2% mole fraction of these compounds, ideally <0.2%.

A further benefit derived from maximising the content of pentabromo ethylbenzene (solid at 20°C) at the expense of the tetrabromo ethylbenzenes (also solid at 20°C) is that a greater density can be achieved (S.G >2.1) whilst the product remains liquid at -10°C.

Yet another advantage of the process is that by altering the proportions of the reactants, the properties of the final reaction product can be varied, thus allowing the density to be maximised for a given fluidity requirement.

Preferably, the overall product from the second stage (b) contains 60-95 mole per cent of di- and tribromo- derivatives, with the tribromo- derivatives predominating, and 40-5 mole per cent of pentabromo derivative, expressed as a mole per cent of the total of di-, tri- and penta- bromo derivatives, and excluding impurities.

It is a further surprising feature of this invention that the bimodal mixture as prepared by the above process has superior physical properties (e.g., as shown by improved liquid stability at -10°C) compared with an equivalent mixture prepared by blending a mixture of di- and tribromo derivatives with a commercially available pentabromo derivative.

Suitable bromination catalysts include Lewis acids (e.g. ferric chloride and bromide, gallium bromide and aluminium chloride), Bronsted acids (e.g. trifluoroacetic acid and methyl sulphonic acid), other halogens, and transition metal complexes, optionally in the presence of promoters such as sulphides, alcohols or acids.

As an alternative to molecular bromine as the source of electrophilic bromine, it is also possible to employ interhalogen type bromides (e.g. Cl-Br), or other halogen carriers where the Br is bonded to an O atom (e.g., acyl and alkyl hypobromites); an S atom (e.g. sulphuryl bromide or sulphuryl chlorobromide); or to an N atom (e.g. N-bromo alkylamines and tetra-alkyl ammonium tribromides).

It is also possible to regenerate evolved hydrogen bromide back to bromine or its kinetic equivalent during the aromatic bromination by means of oxybromination using various forms of oxidants (e.g. oxygen, hydrogen peroxide, chlorate salts, or bleaches such as calcium or sodium hypochlorite). The oxybromination reaction can suitably be promoted using phase transfer techniques ( e.g. the use of tetra alkyl ammonium bromides).

However, the preferred catalyst for the process is that derived from reaction between iron metal and bromine. It is particularly important when using ferric bromide as a catalyst that all the iron metal is converted to the higher oxidation state. This can be achieved by sublimation. Any remaining low valent iron (e.g. Fe° metal) is liable to promote biphenyl-forming reactions (probably due to Ullmann type coupling of aryl bromides) and radical bromine atom formation, resulting in undesirable side chain bromination. A further advantage resulting from quantitative iron metal conversion is the prevention of reactor abrasion. The ferric bromide catalyst can be isolated as a dry powder and subsequently mixed with reactants if desired. However, it is preferred that the ferric bromide is prepared (either in the main reactor or otherwise) in an excess of bromine as solvent and conveniently transferred to the reactor as a semi-solution/suspension in bromine. Thus catalysis of the aromatic bromination is partly by homogeneous ferric bromide and partly by heterogeneous ferric bromide crystals.

The pentabromoethylbenzene formed in the first stage is a solid at room temperature, thus it is preferable to conduct this step in the presence of a solvent. Suitable solvents include carbon tetrachloride, dibromoethane, dichloroethane and dibromoethane, but the preferred solvent is an excess of bromine.

An advantage of performing the process in the absence of solvents other than bromine itself, is that the formation of unwanted and unstable side products (e.g. $CCl_4 \rightarrow CCl_3Br$) is avoided and the process is simplified.

The process is preferably carried out in the dark and optionally in the presence of a radical trap (e.g. Galvinoxyl). Moreover, it is important when using ferric bromide as a catalyst to protect the reactants from moisture, and it may be desirable to charge the reactants under a blanket of inert gas.

Preferably the second stage of the process is carried out in the presence of a polar solvent such as nitromethane, acetonitrile or dimethylacetamide in order to moderate the power of the catalyst acidity. USP 4425447 proposes the use of nitromethane to prevent transalkylation reactions during cumene bromination, thus nitromethane (or other polar solvent) can be used in both stages (a) and (b). However, an additional benefit in the present case is the ability to moderate the power of the catalyst at the onset of mixing the product of stage (a) with the aromatic compound in stage (b) and also at the end of stage (b). Thus it is possible to reduce the conversion of the aromatic compound to higher brominated compounds such as the tetra- and pentabromo- derivatives in stage (b).

The process may suitably be operated at ambient temperature (25°C), though lower and higher temperatures may be employed if so desired. Atmospheric, subatmospheric or elevated pressures may be employed.

The rate of reaction will depend on the amount of catalyst used, the rate of addition of reactants and the temperature. It is preferred that 0.2 to 1% by wt, most preferably 0.5% by wt, of iron powder (based upon total mass of aromatic compound) is used to prepare the ferric bromide catalyst when the latter is to be employed.

It is preferable to perform the process batchwise with continuous agitation.

It may also be desirable to trap the evolved hydrogen bromide gas external to the reactor and convert it to bromine via oxybromination and thus recycle the bromine.

Following the bromination reaction, the product can be purified by aqueous washing, optionally in the presence of sodium metabisulphite and/or sodium hydroxide. In addition, it may be desirable to remove the excess bromine and hydrogen bromide under vacuum. Following neutralisation, it is not necessary to distil the product. However, it is desirable to dry the product using calcium chloride, magnesium sulphate, or activated solids such as molecular sieves.

If a product with very good thermal stability (e.g. at 200°C) is desired then it may be necessary to treat the brominated aromatic compounds with activated solids (e.g. alumina or carbon) at elevated temperature in a stream of dry nitrogen.

The invention is illustrated by reference to the following example.

### Example

### Catalyst

Iron powder (0.5g) was mixed at 25°C with 22.15g of AR grade bromine (about 15 mole equivalent based on iron) under an atmosphere of dry nitrogen in a flat bottomed glass flask of 500 ml capacity. The flask was agitated by a glass overhead stirrer passing through an air-tight gland. The flask was suspended by means of a retort stand and clamp such that the vessel was evenly spaced within the confines of a 5 litre ultrasonic bath supplied by Kerry Ultrasonics Ltd. The bath had a 200 W output and contained 4 litres of deionised water.

The reaction was maintained for about 15 hours. A fine suspension/solution of iron(III) bromide in bromine was obtained, the particle size of the solid particles being less than 10 micron.

The ferric bromide product was transferred in bromine solution directly to the reactor.

### Stage (a) Bromination. Pentabromo-ethylbenzene Formation

8.9 mmoles of iron bromide in 20g of bromine was charged into a reactor (1 litre) containing 130g of bromine and stirred continuously at 25°C via a magnetic follower.
Time = 5 mins.
Next, 11g of ethylbenzene/nitromethane mixture (preformed from 10.9g ethylbenzene and 0.1g of nitromethane) was added dropwise over 1 hour and left to react for a further 1 hour during which the temperature increased from 25° to 27°C. The purpose of the nitromethane was to reduce transalkylation. The evolved hydrogen bromide gas was trapped with a sodium hydroxide (2M) scrubber.
Total time = 2 hr 5 min.

### Stage (b) Bromination. Di- and Tri-bromoethylbenzene Formation

Nitromethane (0.5g) was added to the product from stage (a) over 5 mins., followed by the addition of ethylbenzene (95g) as quickly as possible (with respect to exotherm control) in less than 15 mins. In stage (b) the nitromethane had the additional function of modifying the catalyst.
Total time = 2 hr 25 min.
Bromine (356g) was then charged at a rate of about 100g/hr. Over the first hour, the temperature increased to 27°C and gradually dropped over the following 2.5 hr to 24°C.
Total time = 5 hr 55 min.
The reaction mixture was then stirred for an additional 2 hour period before being run off into a work-up flask.
Total time = 7 hr 55 min.

### Reaction Monitoring

Progress of the reaction was monitored using capillary glc. Samples taken at hourly intervals were neutralised with sodium metabisulphite solution (10% w/w) and extracted into tetrachloromethane.

### Work-Up Procedure

The reaction mixture was firstly mixed with 100 ml water (in 1 litre conical flask) with vigorous stirring. Following separation of the waste aqueous layer, the final traces of bromine were removed using 100 ml of a 10% w/w sodium metabisulphite solution, separated again, and treated with 100 ml of 10% w/w sodium hydroxide solution and more vigorous stirring. The dense oil was then finally washed with three 100 ml aliquots of water and dried over 5g of magnesium sulphate. Weight of crude product = 330g, expected maximum = 350g (97% bromine utilisation).

Product Stabilisation

330g of dense oil was mixed with 15g of alumina and the resultant slurry was stirred at 140°C for 3.5h with a gentle purge of dry $N_2$ to remove any hydrogen bromide fumes. The treated oil was then filtered (while still hot ca. 100°C) over a Buchner funnel and gave 314g of clear dense oil product (95% recovery).

Product Characterisation/Properties

(a) Composition

Glc analysis indicated that the major product composition contained dibromoethylbenzenes (mixture of isomers) 20 mole %; tribromoethylbenzenes (2 isomers) 70 mole %; and pentabromoethyl- benzene 10 mole %. (By weight these figures represent 15%, 70% and 15% respectively). Very small quantities of monobromoethylbenzene (0.5 mole %) and tetrabromoethylbenzene (1.0 mole %) were also present and less than 0.1 mole % biphenyls.

$13C_{nmr}$ confirmed the glc product distribution. No side chain bromination was detected. The most prominent tribromo isomer was 2,4,5-tribromoethylbenzene ( 63 mole %).

(b) Specific Gravity

Specific gravity was measured as 2.1 ± 0.01 at 21°C.

(c) Low Temperature Stability

The oil remained homogeneous at -10°C for a period in excess of 21 days.

(d) High Temperature Thermal Stability

No exotherm could be detected by microcalorimetry of an aereated sample (air bubbled through for 4h) held at 200°C for 2 days. The appearance did not change and pH paper detected no evolution of gaseous hydrogen bromide following thermal treatment.

Claims

1. A two-stage process for the bromination of an aromatic hydrocarbon characterised by the fact that the process comprises:
(a) initially reacting the aromatic compound with bromine in a molar ratio of bromine to aromatic compound in the range 30:1 to 5:1, in the presence of a bromination catalyst to convert the compound substantially to the pentabromo-derivative and
(b) subsequently reacting, in the presence of the products from stage (a), a further quantity of aromatic compound in a ratio to the initial quantity in the range 100:1 to 0.1:1, with a further quantity of bromine in a molar ratio of bromine to aromatic compound in the range 10:1 to 1:1, in the presence of the bromination catalyst, for a time sufficient to convert the reactants of stage (b) to tribromo derivatives or a mixture of tribromo and dibromo derivatives in which tribromo-derivatives predominate.

2. A two-stage process for the bromination of an aromatic hydrocarbon as claimed in claim 1 wherein in the first stage, the ratio of bromine to aromatic compound is from 10:1 to 7:1, and in the second stage, the ratio of the further quantity of aromatic compound to the initial quantity is from 20:1 to 5:1 and the ratio of the further quantity of bromine to aromatic compound is from 5:1 to 2:1.

3. A two-stage process for the bromination of an aromatic hydrocarbon as claimed in claim 1 or 2 wherein the reaction product of stage (a) is added to the second portion of the aromatic compound contained in a second reactor and then more bromine is added.

4. A two-stage process for the bromination of an aromatic hydrocarbon as claimed in claim 1, 2 or 3 wherein, in the second stage, all the further quantity of aromatic compound is added before any of the further quantity of bromine.

5. A two-stage process for the bromination of an aromatic hydrocarbon as claimed in any of claims 1 to 4 wherein the aromatic hydrocarbon is an alkyl aromatic hydrocarbon.

6. A two-stage process for the bromination of an aromatic hydrocarbon as claimed in claim 5 wherein the alkyl aromatic hydrocarbon is ethyl benzene or cumene.

7. A two-stage process for the bromination of an aromatic hydrocarbon as claimed in any of claims 1 to 6 wherein the bromination catalyst is a Lewis acid.

8. A two-stage process for the bromination of an aromatic hydrocarbon as claimed in claim 7 wherein the Lewis acid is iron bromide.

9. A two-stage process for the bromination of an aromatic hydrocarbon as claimed in claim 8 wherein the iron bromide is derived from reaction between iron metal and bromine.

10. A two-stage process for the bromination of an aromatic hydrocarbon as claimed in any of claims 1 to 9 wherein both stages are carried out in the presence of a solvent.

11. A two-stage process for the bromination of an aromatic hydrocarbon as claimed in claim 10 wherein the solvent is an excess of bromine.

12. A mixture of brominated aromatic hydrocarbons characterised by the fact that it consists essentially of from 40-95 mole per cent of tri-bromo derivatives or di- and tri-bromo derivatives with the tri-bromo derivatives predominating, from 40-5 mole per cent of penta-bromo derivative, and not more than 2 mole per cent of tetra-bromo derivatives.

13. A mixture of brominated aromatic hydrocarbons as claimed in claim 12 whenever produced by a process as claimed in any of claims 1 to 11.

14. A solids-free, non-aqueous well bore fluid characterised by the fact that it comprises a mixture of brominated aromatic hydrocarbons having a composition as claimed in claim 12 or 13 or having been produced by a process as claimed in any of claims 1 to 11.

| | DOCUMENTS CONSIDERED TO BE RELEVANT | | EP 89300845.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | EP - A2 - 0 247 801 (BRITISH PETROLEUM) <br> * Examples 5,6; claims 1,2,12,13,22 * | 1,5-10,12-14 | C 07 C 25/02 <br> C 07 C 17/12 <br> C 09 K 7/06 |
| A | DE - A1 - 3 519 221 (HYDROCOR) <br> * Page 7, line 5 - page 8, line 6; page 10, line 17 - page 11, line 16; claims 1-3,7 * | 1,5,7-9,12 | |
| A | US - A - 3 305 487 (HITCHCOCK et al.) <br> * Column 3, lines 14-19; claim 1 * | 1,5-9,13 | |

TECHNICAL FIELDS SEARCHED (Int. Cl.4)

C 07 C 25/00

C 07 C 17/00

C 09 K 7/00

The present search report has been drawn up for all claims

| Place of search | Date of completion of the search | Examiner |
|---|---|---|
| VIENNA | 12-05-1989 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82